# EUROPÄISCHE PATENTSCHRIFT

(11) **EP 1 067 374 B1**
(45) Veröffentlichungstag und Bekanntmachung des Hinweises auf die Patenterteilung: **27.03.2013**
(21) Anmeldenummer: 00110821.6
(22) Anmeldetag: 22.05.2000
(51) Int. Cl.: G01N 1/28, B01L 3/02, C12M 1/26

(54) **Vorrichtung zur Mikro-Dissektion von Gewebe**
Device for the microdissection of tissue
Dispositif pour la microdissection d'un tissu

(30) Priorität: 09.07.1999 DE 19932032
(43) Veröffentlichungstag der Anmeldung: 10.01.2001
(73) Patentinhaber: EPPENDORF AG, 22339 Hamburg (DE)
(72) Erfinder: Hofmeier, Gerhard, Dr. rer. nat., 22399 Hamburg (DE); Schmidt-Rabenau, Hartmut, Dipl.-Ing., 22339 Hamburg (DE); Niendorf, Axel, Prof. Dr. med., 22587 Hamburg (DE)
(74) Vertreter: Emmel, Thomas

(56) Entgegenhaltungen:
- WO-A-99/28725
- DE-C- 19 714 987
- US-A- 4 425 115
- US-A- 4 428 748
- US-A- 4 634 420
- US-A- 5 229 679
- US-A- 5 877 008

## Beschreibung

Die Erfindung bezieht sich auf eine Vorrichtung zur Mikro-Dissektion von Gewebe nach dem Oberbegriff des Anspruchs 1.

Gattungsgemäße Vorrichtungen werden z.B. in Verbindung mit der molekularbiologischen Tumordiagnostik eingesetzt, bei der die DNA oder RNA von Zellen in Tumorgewebsschnitten analysiert wird. Das Vorhandensein z.B. bestimmter DNA-Sequenzen läßt Rückschlüsse auf die Art des Tumors und (soweit hierüber bereits statistische Daten vorliegen) über den Verlauf der Krankheit zu.

Allerdings enthalten Tumorgewebsschnitte nicht ausschließlich Tumorzellen. In aller Regel sind in solchen Schnitten einzelne Bereiche verdächtiger (d.h. tumoröser) Zellen in ansonsten gesundes Zellgewebe eingebettet. Wird der gesamte Schnitt molekularbiologisch untersucht, so besteht die Gefahr, daß die DNA der Tumorzellen mengenmäßig gegenüber der DNA der anderen Zellen soweit zurücktritt, daß ein Nachweis von bestimmten Tumorzell-DNA-Sequenzen nicht mehr oder nicht mehr mit ausreichender Sicherheit möglich ist.

Man ist daher dazu übergegangen, aus solchen Tumorgewebsschnitten die unter dem Mikroskop identifizierbaren tumorösen Gewebsbereiche bzw. Zellen mittels Mikro-Dissektion herauszulösen und nur diese weiter aufzuarbeiten.

Es sind z.Zt. unterschiedliche Techniken zur Mikro-Dissektion von Gewebe bekannt. Eine Möglichkeit besteht z.B. darin, den interessierenden Gewebebereich mittels UV-Laserstrahlung von dem übrigen Gewebe des Schnittes zu trennen und dann mittels z.B. Aspiration zur weiteren Aufarbeitung zu entfernen. Eine weitere bekannte Möglichkeit besteht darin, eine Spezialfolie auf dem Schnitt anzuordnen und diese mittels Infrarotlaserstrahlung im Bereich des Gewebebereiches mit dessen Oberfläche "zu verkleben". Der Gewebebereich haftet nach Verkleben so fest an der Folie, dass er zusammen mit dieser von dem Gewebeschnitt entfernt werden kann. Beide Verfahren sind allerdings relativ aufwendig.

Bekannte Vorrichtungen zur Mikro- und auch Makro-Dissektion, unterscheiden sich des Weiteren von der erfindungsgemäßen Vorrichtung durch die Art der Bewegung, die eine Nadel ausführt und die Steuerung der Nadel. In der WO 99/28725 wird beispielsweise eine automatisierte Mikromanipulationsvorrichtung beschrieben, die eine Kugelrollspindel (siehe Seite 22, Zeilen 9-11) verwendet um die Bewegung der Nadel zu steuern. In der US 4,425,115 wird ein chirugisches Gerät zur Makro-Dissektion, insbesondere zur Fragmentierung und Absaugung von Geweben beschrieben, bei dem Vibrationen über Ultraschall erzeugt werden. Ein solches gerät eignet sich jedoch nicht zur Mikro-Dissektion. Die US 5,877,008 offenbart ein elektromechanisches Mikro-Injektionsgerät für Blastocyten, das einen Piezoelement Stapel umfasst. Mit diesem Injektionsgerät wird eine lineare Stoßbewegung einer Nadel gesteuert. Aus der US 5,229,679 ist ebenfalls eine lineare Bewegung einer Nadel eines Mikromanipulators, der ein piezoelektrischen/elektrostriktiven Element umfasst, bekannt. Bei einer anderen gattungsgemässen Vorrichtung wird das interessierende Gewebe mit dem ggf. angeschliffenen Ende einer Stahlkanüle, die mit einem üblichen Manipulator bewegt wird, aus dem Schnitt herausgekratzt und die abgetrennten Gewebebereiche mittels einer Aspirations- oder Klebkanüle zur weiteren Aufarbeitung entfernt. Bekannt ist auch, anstelle einer Stahlkanüle eine fein ausgezogene Glaskanüle zu verwenden, deren Spitze ggf. gebrochen oder auf andere Weise geschärft ist. Das Arbeiten mit den beschriebenen "Kratz"-kanülen stellt sich sehr mühselig dar. Es besteht darüber hinaus die Gefahr, dass der Schnitt unkontrolliert zerreisst.

Ausgehend hiervon ist es die Aufgabe der Erfindung, eine Vorrichtung zur Mikro-Dissektion zu schaffen, die eine bequemere und sicherere Gewebetrennung ermöglicht und die darüber hinaus relativ kostengünstig zu verwirklichen ist.

Gelöst wird diese Aufgabe mit einer Vorrichtung, die die kennzeichnenden Merkmale des Anspruches 1 aufweist. Vorteilhafte Ausgestaltungen der Erfindung sind in den Unteransprüchen angegeben.

Die erfindungsgemäße Vorrichtung benutzt zur Mikro-Dissektion, wie aus dem Stand der Technik bekannt, eine feine Nadel, die in einer bezüglich der 3 Raumachsen verfahrbaren Aufnahme eines üblichen Manipulators angeordnet ist.

In seiner einfachsten Ausgestaltung weist ein in der erfindungsgemäßen Vorrichtung einsetzbarer Manipulator nur die bezüglich aller 3 Raumachsen verfahrbare Aufnahme auf. Üblicherweise aber nicht zwingend ist die verfahrbare Aufnahme mit einem Mikroskop verbunden dergestalt, daß mit dem freien Ende eines in der Aufnahme gehaltenen Instruments an einem auf dem Mikroskoptisch angeordneten Objekt unter optischer Kontrolle gearbeitet werden kann.

Erfindungsgemäß ist vorgesehen, daß die zur Mikro-Dissektion eingesetzte Nadel mit einem Schwingantrieb gekoppelt ist, der die Nadel in longitudinale und/oder transversale Schwingungen mit vorwählbarer Frequenz und Amplitude versetzen kann.

Im Betrieb wird der freie Endbereich der schwingenden Nadel mit der verfahrbaren Aufnahme gezielt in Kontakt mit ausgewählten Bereichen des Gewebes gebracht und zertrennt bzw. zertrümmert diese.

Abhängig von der Schwingungsrichtung der Nadel kann die erfindungsgemäße Vorrichtung grundsätzlich in zwei unterschiedlichen Varianten betrieben werden.

In der ersten Variante ist vorgesehen, daß die Nadel von dem Schwingantrieb in longitudinale Schwingungen versetzt wird, dann zur Dissektion entlang des Randes des interessierenden Gewebebereichs geführt wird und diesen als Ganzes aus dem Gewebeschnitt herauslöst. Bei dieser Variante der Erfindung wird die Frequenz der Nadel so gewählt, daß möglichst nur longitudinale Schwingungen auftreten und transversale Resonanzschwingungen vermieden werden.

In der zweiten Variante der Erfindung werden Nadel und Schwingantrieb so gekoppelt, daß die Nadel transversal schwingt. In diesem Fall wird der Gewebebereich nicht entlang einer Linie freigeschnitten. Es wird vielmehr mit dem Ende der transversal schwingenden Nadel der interessierende Gewebebereich stückweise flächig herausgekratzt. Mit dieser Variante lassen sich insbesondere mehrere kleine Gewebebereich schneller aus einem Gewebeschnitt herauslösen, als dies mit der ersten Variante möglich ist.

Beide Variante lassen sich ohne größere Probleme durch entsprechenden Aufbau der erfindungsgemäßen Vorrichtung verwirklichen.

Die erste und zweite Variante setzen dabei allerdings nicht zwingend eine unterschiedlich gerichtete Kopplung von Schwingantrieb und Nadel voraus. Es ist auch denkbar, bei einer Vorrichtung, in der die Nadel in longitudinale Schwingungen versetzt wird, die Frequenz gezielt so zu wählen, daß neben einer longitudinalen Grundschwingung (mit ggf. sehr geringer Amplitude) im Spitzenbereich der Nadel transversale Resonanzschwingungen auftreten, womit dann ebenfalls ein Betrieb in der oben beschriebenen zweiten Variante möglich ist.

Bei der ersten Variante kann das in einem Stück abgetrennte Gewebe z.B. mittels Aspiration oder auch mittels einer Klebekanüle aus dem Schnitt entfernt werden. Bei der zweiten Variante wird vorzugsweise die über dem Gewebeschnitt befindliche Flüssigkeit zusammen mit den herausgelösten Gewebebruchstücken von dem Schnitt abgesaugt und gesammelt.

Bei entsprechend eingestellter Schwingfrequenz und Amplitude ist die Nadel bei beiden Varianten ohne weiteres in Lage, mit ihrer Spitze in übliches Gewebe einzudringen und dieses zu zertrümmern bzw. zerteilen.

Da Gewebeschnitte in aller Regel sehr dünn sind, reicht bei der ersten Variante in den meisten Fällen eine Amplitude, die im Mikrometerbereich liegt. Wird die Vorrichtung in der zweiten Variante betrieben, so sollte eine Amplitude für die Transversalschwingung der Nadel gewählt werden, die deutlich unterhalb des Durchmessers des herauszutrennenden Gewebebereichs liegt, um eine möglichst saubere Präparation zu ermöglichen.

Die Frequenz sollte vorzugsweise, um akustische Beeinträchtigungen des Bedieners zu vermeiden, außerhalb des Hörbereiches, also z.B. im Ultraschallbereich gewählt werden. Grundsätzlich reicht aber eine Frequenz aus, bei der sichergestellt ist, daß der Spitzenbereich der Nadel so schnell in dem Gewebe schwingt, daß keine die Dissektion störende mechanische Beanspruchung der direkt angrenzenden Gewebebereiche eintritt. Mit anderen Worten die Frequenz sollte so eingestellt werden, daß die nur seitlich in Kontakt mit der bewegten Nadel kommenden Gewebebereiche nicht oder nur unwesentlich von dieser mitbewegt werden. Auf diese Weise ist sichergestellt, daß tatsächlich nur der gerade gewünschte Gewebebereich von der Nadelspitze zertrümmert bzw. zertrennt wird, und ein Abreißen bzw. Ablösen benachbarter Bereiche vermieden wird.

Bei der Nadel kann es sich um eine vorzugsweise geätzte Stahlnadel handeln. Die Nadel kann aber auch aus Glas, Hartmetall oder auch Wolfram bestehen. Es ist auch möglich, die Nadel an ihrem Ende mit einem Schliff zu versehen. Es hat sich allerdings herausgestellt, daß die distale Kontur der Nadel für die Umsetzung der Erfindung relativ unerheblich ist. Der Begriff Nadel ist daher im Rahmen der Erfindung nicht zu eng auszulegen. Darunter soll im wesentlichen jedes feine langgestreckte Instrument fallen mit einem Endbereich, dessen Abmessungen und Kontur ein Eindringen in das zu untersuchende Gewebe ermöglichen. Insbesondere bei der oben erwähnten zweiten Variante muß die Nadel nicht zwingend in einer Spitze auslaufen, sondern kann auch eine transversale Abschlußkante aufweisen, die ggf. ein leichteres Herausschaben der Gewebstücke ermöglicht.

Zur Erzeugung der Schwingung kann ein üblicher Schwingantrieb verwendet werden. Denkbar ist z.B., daß der Antrieb elektrodynamisch erfolgt. Vorzugsweise wird allerdings zur Erzeugung der Schwingung ein Piezo-Element eingesetzt.

Der Schwingantrieb wird bevorzugt an der in den drei Raumachsen verfahrbaren Aufnahme angeordnet, und die Nadel dann in Abhängigkeit mit der gewünschten Schwingungsrichtung entsprechend mit dem Antrieb gekoppelt.

Eine weitere Ausgestaltung der Erfindung betrifft die optimale Positionierung der Nadel während der Dissektion. Es versteht sich, daß die Nadel bei beiden Varianten mit ihrer Spitze möglichst tief in das Gewebe eindringen muß, wobei allerdings vermieden werden sollte, daß die Spitze die Oberfläche des den Schnitt tragenden in aller Regel aus Glas bestehenden Objekträgers berührt. In diesem Zusammenhang sieht daher eine Ausgestaltung einen Sensor in der erfindungsgemäßen Vorrichtung vor, der in der Lage ist, die optimale Position der Nadel zu ermitteln. Denkbar ist z.B., daß es sich um einen in dem Schwingantrieb angeordneten Schwingungssensor handelt. Bei Einsatz eines solchen Sensors würde die Nadel zunächst so positioniert, daß sie mit ihrer Spitze den Objektträger berührt und dann soweit nach oben verfahren, bis der Schwingungssensor freie Schwingung anzeigt, was dann einer optimale Position entspräche. Eine andere Möglichkeit wäre, z.B. im Bereich der den Gewebeschnitt aufnehmenden Auflage einen akustischen Sensor anzuordnen, der die Berührung des Objektträgers durch die Nadelspitze registriert und dann entsprechend die Position einstellt.

Wie oben ausgeführt, arbeitet die erfindungsgemäße Vorrichtung in aller Regel mit einem Manipulator, der im Mindestfall eine bezüglich der drei Raumachsen verfahrbare Aufnahme für die Nadel bereitstellt. Es ist allerdings denkbar, daß diese Aufnahme mit unterschiedlichen zellbiologischen Instrumenten gekoppelt werden kann. Daher richtet sich die Erfindung nicht nur auf die oben beschriebene Vorrichtung in ihrer Gesamtheit. Vielmehr soll die Erfindung auch eine Einheit aus Nadel und Schwingantrieb abdecken, die ggf. als auswechselbares Zubehörteil für übliche in der Zellbiologie einsetzbare Manipulatoren dienen kann.

Im folgenden soll die Erfindung anhand einer Abbildung näher erläutert werden.

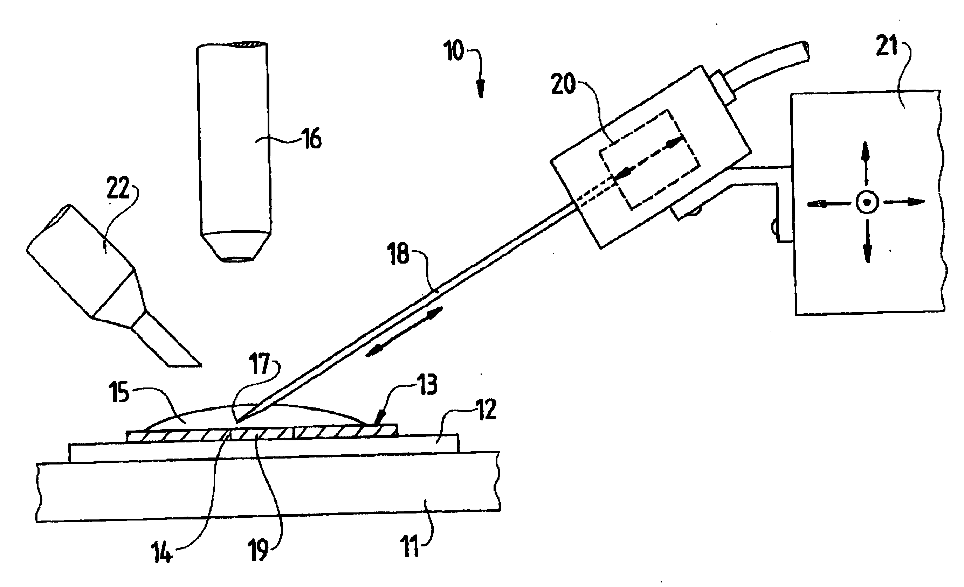

Die Abbildung zeigt eine erfindungsgemäße Vorrichtung 10 zur Mikro-Dissektion. Die Vorrichtung beinhaltet eine Auflage 11 für einen Objektträger 12. auf dem ein Gewebeschnitt 13 angeordnet ist. Aus dem Gewebeschnitt 13 soll ein Bereich 19 entlang einer Linie 14 mittels Mikro-Dissektion herausgetrennt werden. Der Schnitt 13 ist partiell insbesondere in dem durch die Linie 14 begrenzten Bereich 19 mit einer Flüssigkeit 15 überschichtet. Bevorzugt wird eine Flüssigkeit gewählt, die einerseits die erforderliche optische Durchlässigkeit besitzt und andererseits z.B. Substanzen enthält, die im Hinblich auf die nachfolgende Aufarbeitung gezielt ausgewählt sind. Denkbar ist z.B. in der Flüssigkeit RNase-hemmende Substanzen wie chaotrope Salze oder dergleichen vorzusehen. Bei der Flüssigkeit kann es sich natürlich auch um destilliertes Wasser oder Kochsalzlösung oder auch organische Lösungsmittel handeln, um nur einige weitere mögliche Beispiele zu nennen.

Oberhalb der Auflage 11 ist ein Mikroskop angeordnet, von dem in dieser Abbildung nur ein Objektiv 16 dargestellt ist.

Erfindungsgemäß ist zur Mikro-Dissektion des von der Linie 14 begrenzten Gewebebereichs 19 eine mit einer feinen Spitze 17 ausgestattete Nadel 18 vorgesehen, die mit einem nur schematisch angedeuteten Schwingantrieb 20 gekoppelt ist, der die Nadel 18 in Pfeilrichtung in longitudinale Schwingungen versetzt. Der Schwingantrieb 20 ist seinerseits an einer Aufnahme 21 angeordnet, die bezüglich der drei Raumachsen verfahrbar ist.

Mittels der verfahrbaren Aufnahme 21 wird die Nadelspitze 17 so in bzw. an dem Rand 14 des herauszulösenden Gewebebereiches 19 positioniert, daß bei Schwingung eine Durchtrennung des Gewebes eintritt. Die Nadelspitze 17 muß dann lediglich mit der Aufnahme 21 entlang des Randes 14 des Gewebebereiches 19 geführt werden, wodurch dieser dann von dem übrigen Gewebe getrennt wird.

Der freigelegte Gewebebereich 19 kann dann mittels einer nur schematisch angedeuteten Aspirationskapillare 22, die z.B. ebenfalls in einer verfahrbaren Aufnahme angeordnet sein kann aus dem Schnitt 13 zur weiteren Verarbeitung entfernt werden.

Es ist dabei relativ unerheblich, in welchem Winkel die Nadel zu dem Gewebe angeordnet wird. Als vorteilhaft hat sich ein Winkel von ca. 45° - 60° erwiesen. Andere Winkel bringen jedoch auch das gewünschte Ergebnis, wobei möglicherweise nur eine etwas längere Bearbeitungszeit in Kauf genommen werden muß.

Wie oben ausgeführt kann die exakte Positionierung der schwingenden Nadelspitze 17 mittels der Aufnahme 21 über einen Sensor automatisiert werden. Dieser Sensor könnte z.B. mit dem Schwingantrieb 20 gekoppelt sein oder aber auch auf der Auflage 11 positioniert werden.

Die Abbildung zeigt die oben angesprochene erste Variante der Erfindung, bei der der Gewebereich 19 in einem Stück aus dem gezeigten Gewebeschnitt 13 herausgelöst wird. Bei dieser Variante wird bevorzugt die Schwingfrequenz der Nadel so eingestellt, daß keine Transversalschwingungen auftreten. Nur so läßt sich ein möglichst schmaler Schnitt entlang der den Gewebebereich 19 begrenzenden Linie 14 durch das Gewebe führen.

Denkbar ist natürlich auch, die erfindungsgemäße Vorrichtung in einer anderen Ausführung mit einer transversal schwingend angetriebenen Nadel vorzusehen bzw., die Frequenz bei der in der Abbildung dargestellten Vorrichtung so einzustellen, daß neben den primär erzeugten longitudinalen Schwingungen auch transversale Resonanzschwingungen im Spitzenbereich der Nadel auftreten. Geeignet ist diese zweite Betriebsvariante insbesondere für die Dissektion in Gewebeschnitten, die mehrere kleine verdächtige Gewebebereiche enthalten. Diese Gewebebereiche lassen sich mit der transversal schwingenden Spitze bequem stückweise aus dem Schnitt herauslösen, wobei sich die Stücke in der Flüssigkeit 15 sammeln und mit dieser in einfacher Weise mit der Kapillare 22 abgesaugt und aufgefangen werden können.

In beiden Varianten stellt die Erfindung eine Vorrichtung bereit, mit der in einfacher und kostengünstiger Weise eine Mikro-Dissektion durchgeführt werden kann.

## Patentansprüche

1. Vorrichtung zur Mikro-Dissektion (10) von in Form eines flächigen Schnitts auf einem Trager fixierten Gewebe (13) mit einer feinen Nadel (18), die in einer bezüglich der drei Raumachsen verfahrbaren Aufnahme (21) angeordnet ist und deren Spitze (17) mit der Aufnahme relativ zu dem zu trennenden Gewebe bewegbar ist, **dadurch gekennzeichnet, dass** die Nadel (18) mit einem Schwingantrieb (20) gekoppelt ist, der die Nadel in longitudinale und/oder transversale Schwingungen mit vorwählbarer Amplitude und Frequenz versetzt.

2. Vorrichtung nach Anspruch 1, **dadurch gekennzeichnet, dass** die Frequenz im Ultraschallbereich liegt.

3. Vorrichtung nach Anspruch 1, **dadurch gekennzeichnet, dass** Schwingantrieb (20) und Nadel (18) zur Erzeugung einer longitudinalen Schwingung gekoppelt sind, wobei die Frequenz im Betrieb so gewählt ist, dass zusätzlich zu den longitudinalen Schwingungen auch transversale Resonanzschwingungen im Bereich der Spitze (17) der Nadel (18) auftreten.

4. Vorrichtung nach einem der Ansprüche 1-3, **dadurch gekennzeichnet, dass** die Nadel insbesondere aus Stahl besteht.

5. Vorrichtung nach einem der Ansprüche 1-4, **dadurch gekennzeichnet, dass** der Schwingantrieb (20) ein Piezo-Element ist.

6. Vorrichtung nach einem der Ansprüche 1-5, **dadurch gekennzeichnet, dass** ein Sensor vorgesehen ist, der die optimale Position der Nadelspitze (17) während des Betriebs ermittelt.

7. Vorrichtung zur Mikromanipulation gemäß einem der Ansprüche 1-6, in der die Einheit aus Schwingantrieb (20) und Nadel (18) ein auswechselbares Zubehörteil ist.

8. Vorrichtung gemäß Anspruch 7, bei der die Einheit aus Schwingantrieb (20) und Nadel (18) mit einem üblichen in der Zellbiologie eingesetzten Manipulator verbunden ist.

## Claims

1. An apparatus for the micro-dissection (10) of tissue (13) secured in form of a flat section to a support, comprising a fine needle (18) which Is arranged on a holder (21) movable In space along three axes and the tip (17) of which is movable by the holder relative to the tissue to be severed, **characterized In that** the needle (18) is coupled to an oscillating drive mechanism (20) which causes the needle to oscillate in longitudinal and/or transverse directions at a predetermined amplitude and frequency.

2. The apparatus of claim 1, **characterized in that** the frequency Is In the ultrasonic range.

3. The apparatus of claim 1, **characterized in that** the oscillating drive mechanism (20) and the needle (18) are coupled for producing a longitudinal oscillation, with the frequency being selected during operation so that, In addition to the longitudinal oscillations, also transverse resonance oscillations occur In the region of the tip (17) of the needle (18).

4. The apparatus of one of the claims 1-3, **characterized in that** the needle Is formed especially of steel.

5. The apparatus of one of the claims 1-4, **characterized in that** the oscillating drive mechanism (20) is a piezo-element.

6. The apparatus of claim 1-5, **characterized In that** a sensor is provided for determining the optimum position of the tip (17) of the needle during operation.

7. Apparatus for micro-manipulation according to one of the claims 1 to 6, in which a unity of oscillating drive mechanism (20) and the needle (18) is an exchangeable accessory.

8. Apparatus according to claim 7, wherein the unit of the oscillating drive mechanism (20) and needle (18) is connected with a conventional manipulator used In cell biology.

## Revendications

1. Dispositif de microdissection (10) d'un tissu (13) fixé sous forme de coupe plane sur un support, muni d'une aiguille fine (18) disposée dans un logement (21) déplaçable selon les trois axes de l'espace et dont la pointe (17) peut être déplacée avec le logement par rapport au tissus à sectionner, **caractérisé en ce que** l'aiguille (18) est couplée à un entraînement oscillant (20) qui fait osciller l'aiguille longitudinalement et/ou transversalement avec une amplitude et à une fréquence pouvant être présélectionnées.

2. Dispositif selon la revendication 1, **caractérisé en ce que** la fréquence est située dans la gamme des ultrasons.

3. Dispositif selon la revendication 1, **caractérisé en ce que** l'entraînement oscillant (20) et l'aiguille (18) sont couplés de façon à produire une oscillation longitudinale, la fréquence de service étant choisie de telle sorte que, outre les oscillations longitudinales, apparaissent également des oscillations transversales de résonance au niveau de la pointe (17) de l'aiguille (18).

4. Dispositif selon l'une des revendications 1 à 3, **caractérisé en ce que** l'aiguille notamment est en acier.

5. Dispositif selon l'une des revendications 1 à 4, **caractérisé en ce que** l'entraînement oscillant (20) est un élément piézoélectrique,

6. Dispositif selon l'une des revendications 1 à 5, **caractérisé en ce qu'**un détecteur est prévu pour déterminer la position optimale de la pointe (17) de l'aiguille pendant le service.

7. Dispositif de micromanipulation selon l'une des revendications 1 à 6, l'entraînement oscillant (20) et l'aiguille (18) constituant une unité formant un accessoire interchangeable.

8. Dispositif selon la revendication 7, l'unité constituée par l'entraînement oscillant (20) et l'aiguille (18) étant reliée à un manipulateur courant utilisé en biologie cellulaire.
